# DEMANDE DE BREVET EUROPEEN

(11) **EP 0 894 865 A2**
(43) Date de publication de la demande: **03.02.1999**
(21) Numéro de dépôt: 98111476.2
(22) Date de dépôt: 21.03.1989
(51) Int. Cl.: C12N 15/82, C12N 5/10, A01H 5/00, A01H 1/04

(54) **Gène chimérique de résistance herbicide**

(30) Priorité: 23.03.1988 FR 8804130
(62) Demande divisionnaire de: 89420101.1
(71) Demandeur: RHONE-POULENC AGRO, 69009 Lyon (FR)
(72) Inventeur: Leroux, Bernard, Chazay 69380, Lozanne (FR); Pelissier, Bernard, 60370 Saint Didier au Mont d'Or (FR); Lebrun, Michel, 34090 Montpellier (FR)
(74) Mandataire: Tetaz, Franck

(57) **Abrégé**

- Gène pour conférer aux plantes la résistance à des herbicides
- Gène chimérique utilisable pour conférer aux plantes une résistance à un herbicide à base de dihalogéno-3,5 hydroxy-4 benzonitrile comprenant au moins un gène codant pour la résistance à cet herbicide, un promoteur étranger et éventuellement une zone signal de polyadénylation, caractérisé en ce que le promoteur provient d'un gène s'exprimant naturellement dans des cellules végétales et choisi dans le groupe comprenant le promoteur de l'ARN 35S du virus de la mosaïque du chou-fleur (CamV 35S) et le promoteur de la petite sous unité (PSU) de la ribulose-1,5-bis phosphatocarboxylase oxygénase (RuBisCO de tournesol (Helianthus annuus)
- Application en agriculture.

## Description

La présente invention concerne un nouveau gène chimérique utilisable pour conférer aux plantes une résistance à un herbicide à base de dihalogéno-3,5 hydroxy-4 benzonitrile,un procédé de transformation de cellulles végétales à l'aide de ce gène et les plantes transformées régénérées à partir de ces cellules.

Il est connu,d'après la demande européenne 229 042, de conférer à des plantes une résistance à un herbicide du type cité ci-dessus, en particulier le dibromo-3,5 hydroxy-4 benzonitrile ou bromoxynil,par introduction dans le génome des plantes d'un gène codant pour une nitrilase spécifique de la dégradation de ces herbicides.Si cette technique donne des résultats intéressants, elle demande à être améliorée pour augmenter les chances de succès et valoriser son potentiel économique,en particulier en ce qui concerne le niveau d'expression dans les plantes et,par conséquent,la qualité de la résistance des plantes à ces herbicides.

Dans la présente description,on entend par "plante"tout organisme multicellulaire différentié capable de photosynthèse et par "cellule végétale" toute cellule issue d'une plante et pouvant constituer des tissus indifférenciés tels que des cals ou des embryons ou des tissus différenciés tels que des parties de plantes ou des plantes ou des semences.

La présente invention a pour but de répondre à ce besoin.

Elle concerne un gène chimérique utilisable pour conférer aux plantes une résistance à un herbicide à base d'un dihalogéno-3,5 hydroxy-4 benzonitrile comprenant au moins un gène codant pour une nitrilase de résistance à cet herbicide,un promoteur étranger et éventuellement une zone signal de polyadénylation,caractérisé en ce que le promoteur provient d'un gène s'exprimant naturellement dans les cellules végétales et choisi dans le groupe comprenant le promoteur dde l'ARN 35 S du virus de la mosaïque du chou-fleur (CamV 35S) et le promoteur de la petite sous-unité(PSU) de la ribulose-1,5-bis phosphate carboxylase oxygénase (RubisCO)du tournesol (Helianthus annuus).

Le promoteur du gène chimérique selon l'invention provient d'un gène s'exprimant naturellement dans les plantes ,c'est à dire soit de type non végétal,par exemple viral comme l'ARN 35 S du virus de la mosaïque du chou-fleur (CamV 35S) ou encore et de préférence de type végétal de plantes monocotylédones ou dicotylédones,en particulier la petite sous unité de la ribulose-I,5-bis phosphate carboxylase oxygénase (RubisCO) de tournesol (Helianthus annuus).On peut utiliser ces promoteurs seuls ou en combinaison.Le choix dépend de la nature de la plante(mono- ou dicotylédone) à transformer.Ainsi il est préféré d'utiliser la petite sous unité RubisCO du tournesol pour la transformation d'une plante dicotylédone.

Chacun de ces promoteurs peut être obtenu comme suit:

### 1) Promoteur de l'ARN 35S du virus de la mosaïque du chou-fleur (CamV 35S):

L'isolement de ce promoteur a été décrit par Odell et al(I985). Un clone(pJO5-2) contenant environ 850 bp en amont du site d'initiation de la transcription a été choisi pour les constructions décrites. Un fragment EcoRI-HindIII a été isolé, les extrémités rendues franches par la polymérase de Klenow ,et cloné dans le vecteur pUC 19 (Yannish-Perron et al,1985) au site HincII. Ce clone a été digéré par action de XbaI et PstI et le fragment obtenu traité par la polymérase de phage T4 afin de rendre les extrémités franches. Ce fragment a été cloné dans pUC19 Cm (Buckley,I985)coupé par SmaI et XbaI et traité à la polymérase de Klenow. Le clone ainsi obtenu a été nommé p RPA-BL I45. Par traitement à la polymérase de Klenow du site AccI 3' terminal et en le ligaturant avec le site EcoRI traité à la polymérase de Klenow des fragments situés en aval de ce promoteur, on reconstitue un site EcoRI et la séquence ainsi obtenue à partir du site d'initiation de la transcription est le suivant :

A C A C G C T G A C A A G C T G A C T C A G C T A G A G
T C G A A T T C
EcoRI

### 2)Le promoteur de la petite sous unité de la ribulose-1,5-bisphosphate carboxylase(RubisCO)de tournesol (Helianthus annuus.

Le gène dont ce promoteur est issu a été isolé par Wacksman et al (I987).Un fragment EcoRI contenant le promoteur de ce gène a été cloné dans mp 18, la partie 3' du promoteur directement en amont du polylinker de ce vecteur. Ce clone a ensuite été linéarisé par BstXI et traité par l'exonucléase Bal31. Le mélange de fragments ainsi obtenu a été traité par SalI puis la polymérase de Klenow et enfin ligaturé à basse concentration d'ADN.Les clones obtenus suite à cette manipulation ont été séquencés et l'un d'entre eux présentant la séquence suivante en aval du site putatif d'initiation de la transcription retenu :

...
...5' A T T G G A T T C 3'...

Un linker Cla I (A T C G A T ) a été introduit au site PstI de ce clone. Ainsi par traitement de ce site ClaI à la polymérase de Klenow et ligature avec le site EcoRI traité à la polymérase de Klenow des fragments qui sont placés en aval de ce promoteur, on reconstitue un site EcoRI et la séquence ainsi obtenue à partir du site d'initiation de la transcription putatif est la suivante:

A T T G G A T T C T C G A C C A T C G A A T T C
Eco RI

Selon un autre aspect de l'invention, le gène chimérique comprend une zone intermédiaire non traduite(linker)entre le gène codant et le promoteur et qui peut être choisie dans le groupe comprenant:
- d'une part le linker de pUC 19 modifié par clonage et ayant la séquence suivante : 
G A A T T C G A G C T C G G T A C C C C A T G G
EcoRI NcoI
- d'autre part la zone non traduite de la petite sous unité de la RubisCO de maïs : cette zone est issue de l'ADNc correspondant au gène décrit par Lebrun et al (1987).C'est un fragment EcoRI-NcoI qui a la séquence suivante : 
G A A T T C C C A G C A A G C A A G C A G C G A G T A C
EcoRI
A T A C A T A C T A G G C A G C C A G G C A G C C A T G
NcoI
- d'autre part la zone non traduite de la petite sous unité de la RubisCO du tournesol:cette zone est issue de l'ADNc isolé par Waksman et Freyssinet (I987). Elle n'a pas été isolée en tant que telle et se trouve toujours préceder le peptide de transit de la RubisCO du tournesol. La séquence est la suivante :

G A A T T C C G A A A G A C A A A G A T T A T C G
Eco RI
T A A T G
Met

Le gène chimérique selon l'invention comprend éventuellement une zone ou site de polyadénylation qui peut être par exemple :
1) Le site de polyadénylation du gène de la nopaline synthase de pTi 37 (Bevan et al,I983). Ce site est contenu dans un fragment MboI de 260bp (Fraley et al,I983, demande de brevet PCT 84/02913) qui a été traité par la polymérase de Klenou et cloné dans le site SmaI de M13mp18 pour introduire des sites BamHI et EcoRI respectivement aux extrémités 5' et 3'. Le site BamHI a été traité à la nucléase de Vigna radiata et cloné au site SalI traité à la polymérase de Klenow de pUC 19.Ce fragment contient maintenant a son extrémité 5' un site HindIII qui peut être ligaturé à celui situé en 3' du gène de nitrilase.
2) Le site de polyadénylation du gène de la petite sous unité de la RubisCO du mais : ce site a été isolé sous forme d'un fragment SmaI-BglII de 540bp du gène décrit par Lebrun et al (I987). Un linker ClaI (ATCGAT) a été introduit au niveau du site SmaI. Après coupure avec ClaI et remplissage à la polymérase de Klenow, ce fragment a été cloné dans pUC19 coupé par PstI puis traité par la polymérase du phage T4 et recoupé par BamHI. Cette manipulation a permis d'introduire un site HindIII en 5' du site de polyadénylation.La séquence obtenue est la suivante:

5' A A G C T T G C A T G C C C G A T G G G C A G .....
HindIII 1/SmaI

Selon un autre aspect de l'invention, le gène chimérique peut comprendre éventuellement et de manière préférée, entre la zone intermédiaire et le gène de la nitrilase, une zone codant pour un peptide de transit choisi dans le groupe comprenant celui de la petite sous unité de la RubisCO de maïs et celui de la petite sous unité de tournesol. Le peptide de transit a pour fonction dans le gène naturel de permettre l'entrée de la petite sous unité de la RubisCO dans le stroma des chloroplastes. Ils doivent diriger de même la nitrilase dans ce compartiment dans le cas où ils sont introduits entre la zone intermédiaire précédemment décrite et le gène de structure de la nitrilase :
1) Peptide de transit de la petite sous unité de la RubisCO du maïs : ce fragment est issu de l'ADNc correspondant au gène décrit par Lebrun et al(I987).C'est un fragment Nco I-SphI de 141pb, le site NcoI recouvrant le codon initiateur de la traduction et le site SphI, le site de coupure du peptide de transit. Par traitement de l'extrémité SphI de ce fragment à la polymérase du phage T4 et en la ligaturant avec l'extrémité NcoI traitée à la polymérase de Klenow du gène de la nitrilase,on reconstitue une séquence permettant l'obtention d'une nitrilase non modifiée dans le stroma des chloroplastes.
2) Peptide de transit de la petite sous unité de la RubisCO du tournesol : ce fragment est issu de l'ADNc isolé par Waksman et Freyssinet (1987).Cette séquence ne possède pas à l'origine de site SphI au niveau du site de coupure du peptide de transit. La séquence est la suivante, à ce niveau :
   5' C A A T G C A T G A* A G 3'

On a substitué par mutagénèse dirigée un C au A marqué d'une astérique créant ainsi un site SphI. Pour effectuer cette manipulation, on a utilisé la méthode de Zoller et Smith (I984). Un fragment EcoRI-SalI de 270pb a été cloné dans MI3mp19am4. Ce vecteur dérivé de M13mp19 possède une mutation ambre dans le gène 4 à la base 5327 et ne peut se multiplier dans les souches ne possédant pas de suppresseur de mutation de ce type. Après purification de la forme monobrin de ce phage recomblnant, trois olinucléotides ont été hybridés en une seule étape. La séquence de ces olinucléotides phosphorylés est la suivante:

SphI
1:5'G T T C A A T G C A T G C A G G T G T G G C C A C 3'
2:5' A A G A G T C T G T C C A T C A C 3'
3:5' G T A A A A C G A C G G C C A G T 3'

Ils permettent respectivement :
1 : la mutation du fragment au niveau du site de coupure du peptide de transit,
2 : la correction de la mutation ambre,
3 : l'amorce de séquence en amont du fragment à mutagénèse.

Après action simultanée de la polymérase de Klenow en présence des quatre nucléotides et de la ligase du phage T4, le mélange obtenu a été transformé dans la souche HB2154 puis étalé sur un tapis de HB2151(Carter et al,I985). Parmi les clones obtenus, ceux possédant un site SphI supplémentaire ont été séquencés afin d'en vérifier la structure et l'un d'entre eux a été utilisé pour la création de gènes chimériques. Au niveau du site de coupure du peptide de transit, la séquence est maintenant la suivante :
5' C A A T G C A T G C

Ce fragment est utilisé de façon identique à celle utilisée pour le fragment codant pour le peptide de transit de la petite sous unité de la RubisCO du maïs.

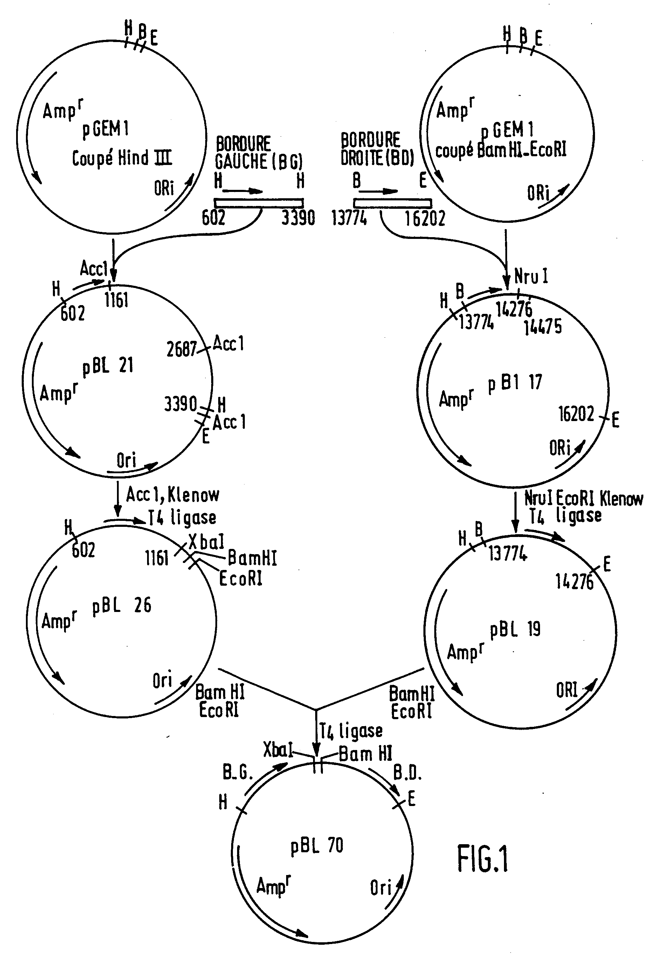

L'assemblage des gènes chimériques s'effectue selon le schéma des figures 1 à 4 avec les éléments précédemment décrits. Les différents gènes ainsi créés ont été placés dans un ou deux types de vecteurs et chaque ensemble a été rapporté à un numéro.Les différents vecteurs ainsi réalisés sont décrits dans le tableau 1 ci-dessous:

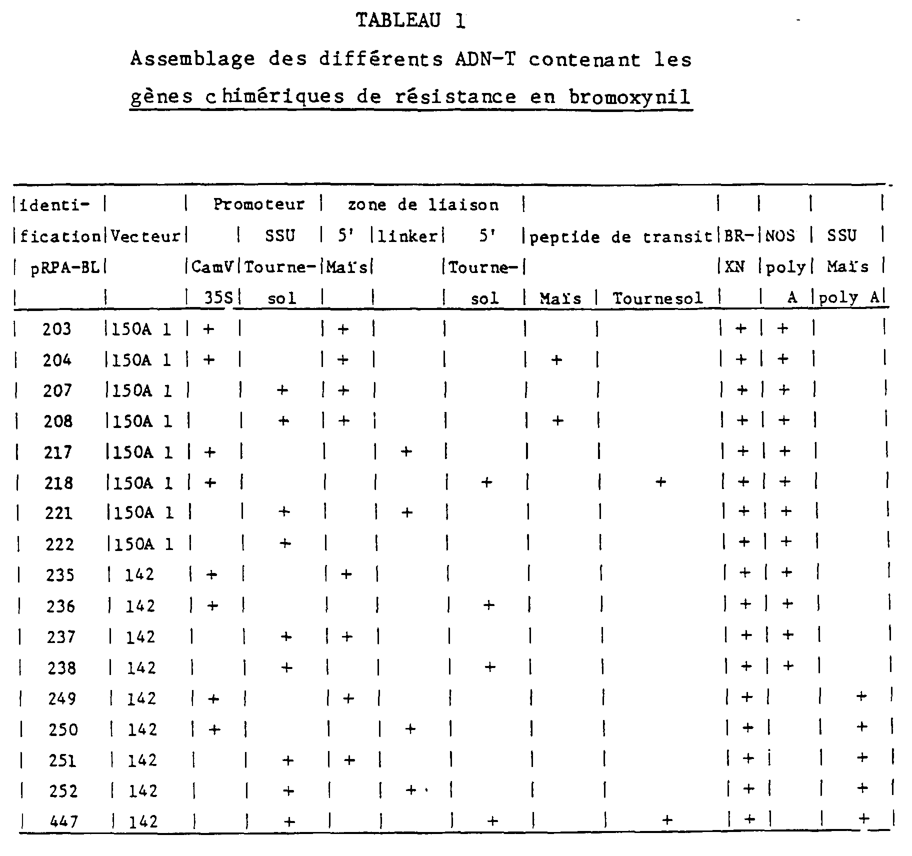

### Vecteurs utilisés:

Les différentes constructions chimériques ayant conduit aux vecteurs pRPA-BL-142 et pRPA-BL-150Aalpha1 représentées aux figures 1 à 4 ont été introduites dans les plantes grâce au système de transfert d'Agrobacterium tumefaciens. Les vecteurs de transfert construits à cet effet ont les caractéristiques suivantes :
- une origine de réplication et de transfert issue de pBR 322,
- un gène de sélection bactérien par exemple la résistance à la gentamicine,
- un site COS issu du phage lambda,
- les deux bordures droite et gauche de l'ADN-T de pTiA6,
- éventuellement un gène de sélection eucaryote comme la résistance à la kanamycine,
- éventuellement un fragment contenant le gène de complémentation lac alpha de pUC18.

### Construction de pRPA-BL-142(Figures I à 4) :

Les bordures droite et gauche (Fig.1)de l'ADN-T de gauche de pTiA6 ont tout d'abord été sous clonées :

### - bordure droite :

un fragment BamHI-EcoRI allant de 13774 à 16202 dans le système de numération de Barker et al (1983) a été cloné dans pGEM 1 (Promega Biotech) aux sites correspondants donnant pBL-17.Ce plasmide a été digéré par NruI (14276 et 14475) et par EcoRI (16202) et traité à la polymérase de Klenow. La ligation des sites NruI et EcoRI rempli régénère un site EcoRI à 14276 et donne le plasmide pBL-19.

### - bordure gauche :

un fragment HindIII allant de 602 à 3390 dans le système de Barker et al (1983) a été clone dans le site correspondant de pGEM 1 donnant pBL-21,dans lequel la bordure gauche est à l'opposé du polylinker. Ce plasmide a été digéré par Acc 1 (1161 et 2687) et traité à la polymérase de Klenow avant d'être ligaturé. Le plasmide résultant,pBL-26 contient un fragment allant de 602 à 1161, inséré entre les sites HindIII et XbaI.

### Création de l'ADN-T :

Par introduction du fragment EcoRI-BamHI de pBL-19 dans les sites correspondants de pBL-26, un ADN-T a été reconstitué possédant les bordures droite et gauche de pTiA6 dans leur orientation naturelle. Le plasmide obtenu est désigné par pBL-70.

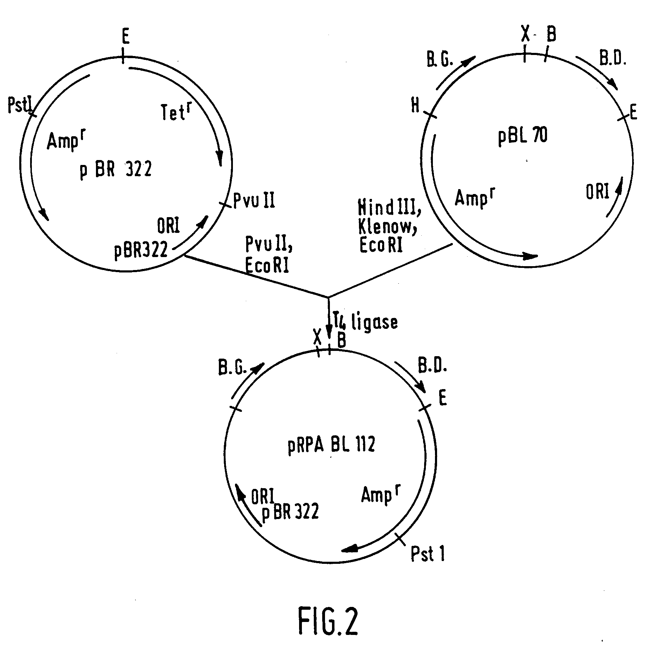

Introduction de l'ADN-T dans pBR 322 (Fig.2) : Après coupure de pBL-70 par HindIII, ce site a été traité par la polymérase de Klenow et le plasmide recoupé par EcoRI. Le fragment obtenu a été cloné dans pBR 322 coupé par PruII-Eco RI.Le clone résultant est désigné par pRPA-BL-112.

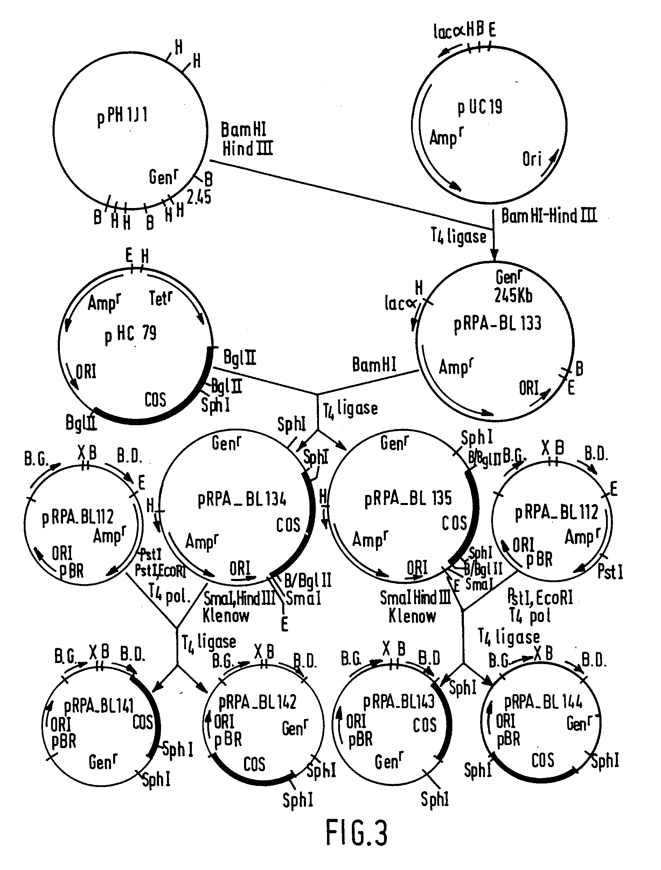

### Clonage d'un gène de résistance à la gentamicine (Fig.3) :

Le gène de résistance à la gentamicine a été obtenu à partir de pPHl JlI (Hirsh and Bringer,I984). Ce plasmide a été digéré par BamHI et HindIII et l'ensemble des fragments clonés dans pUC 19 coupé par les mêmes enzymes.
Après
sélection sur ampicilline + gentamycine, plusieurs clones contenant un fragment de 2,45kpb ont été isolés.Le clone retenu pour la suite des manipulations a été nommé pRPA-BL-133. Au site BamHI de ce clone, on a introduit un fragment BglII de 1,6Kb isolé de pHC 79 (Hohn and Collins,I980) et contenant le site COS du phage lambda. Ce fragment, inséré dans les deux orientations, a permis d'obtenir deux clones pRPA-BL-134 et pRPA-BL-135.

### Obtention d'un vecteur intégratif (Fig.3) :

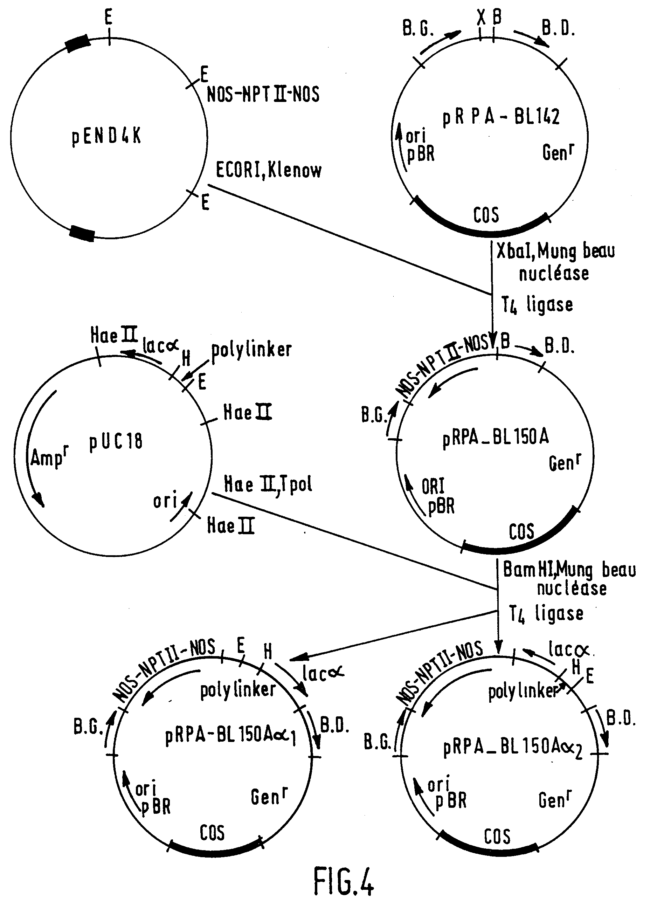

Afin de réunir dans un même vecteur les différentes parties précédemment décrites, les plasmides pRPA-BL-134 et pRPA-BL-135 ont été digérés par SmaI et HindIII et l'insert contenant le gène de résistance à la gentamicine et le site COS du phage lambda traité par la polymérase de Klenow. Le plasmide pRPA-BL-112 a été digéré par PstI et EcoRI et traité par la polymérase du phage T4. Les deux fragments ont été ligaturés et les clones contenant à la fois la résistance à la gentamicine, le site COS, l'ADN-T et l'origine de réplication de pBR 322 ont été retenus. Le plasmide pRPA-BL-134 a donné naissance à pRPA-BL-141 et pRPA-BL-142 et pRPA-BL-135 a donné naissance à pRPA-BL-143 et pRPA-BL-144. C'est pRPA-BL-142 qui a été retenu pour l'introduction des gènes chimériques à transférer dans les plantes. A partir de ce vecteur,une construction contenant un gène marqueur NOS-NPTII-NOS (Fig.4) a été obtenue. Le plasmide pRPA-BL-I42 a été digéré par XbaI et les extrémités réduites par action de la nucléase de Vigna radiata. Par ailleurs pEND4 K (Klee et al,I985)a été digéré par EcoRI et traité par la polymérase de Klenow. Un fragment de I,6 Kpb a été isolé contenant le gène chimérique de résistance à la kanamycine et introduit dans pRPA-BL-142. Le résultat d'une de ces fusions a été nommé pRPA-BL-150 A et a été choisi pour la suite des manipulations. Afin de faciliter le clonage dans ce vecteur, on a introduit au site BamHI traité par la nucléase de Vigna radiata un fragment HacII traité par la polymérase du phage T4 et contenant le gène de complémentation lac alpha isolé de pUC 18 (Yannish-Perron et al,1985). Les deux vecteurs obtenus ont été nommés pRPA-BL-150Aalpha1 et pRPA-BL-150Aalpha2. C'est pRPA-BL-150Aalpha1 qui a servi de base pour l'introduction de gènes dans les plantes.

### Utilisation de pRPA-BL-142 et de pRPA-BL-150Aalpha1

Ces vecteurs ne se maintiennent pas seuls dans Agrobacterium. Pour se maintenir, il leur est nécessaire de s'intégrer par simple recombinaison dans un plasmide résident dans cette bactérie. Cela peut se produire via un des fragments tels que le site qui se trouve sur des cosmides tels que pVK 102 ou autres ou tels que le fragment de pBR 322 pour des plasmides possédant de telles séquences. C'est le cas pour le plasmide Ti de la souche GV3850 (Zambryski et al 1983) qui est aussi un hôte pour pRPA-BL-142 et pRPA-BL-150Aalpha1. Utilisant l'origine de réplication de pBR 322, ces plasmides sont transférés dans Agrobacterium via le système tripartite décrit par Ditte et al (1980).

### Transformation du matériel végétal

Afin de tester l'efficacité de ces gênes chimériques,ceux-ci ont été transférés dans du matériel selon les procédures décrites ci-dessous :

### A - Procédures de transformation

### 1. Tabac

Le vecteur est introduit dans la souche non oncogène d'Agrobacterium EHA 101 (Hood et al 1987) porteuse du cosmide pTVK 291 (Komari et al, 1986).
La technique de transformation est basée sur la procédure d'Horsh et al (1985). La procédure de régénération du tabac industriel PBD6 (provenance SEITA, France) est décrite ci-dessous.
La régénération du tabac PBD6 à partir d'explants foliaires est réalisé sur un milieu de base Murashige et Skoog (MS) comprenant 30 g/l de saccharose. Les explants foliaires sont prélevés sur des plantes cultivées en serre ou in vitro et transformé selon la technique des disques foliaires (Science 1985, vol 227, p.1229-1231 en 3 étapes successives.
La première étape comprend l'induction des pousses sur un MS+30 g de saccharose comprenant 0,05 mg d'ANA et 2 mg/l de BAP pendant 15 jours.
La deuxième étape permet le développement des pousses formées au cours de la lère étape ; elle est faite sur un MS+30 g/l de saccharose et ne comprenant pas d'hormone,pendant 10 jours.
La troisième étape permet l'enracinement des pousses prélevées individuellement. Ce milieu d'enracinement contient les sels, vitamines et sucre dilués de moitié. Il ne contient pas d'hormone. Après 10 à 15 jours, les pousses enracinées sont passées en terre.

### Détermination de la balance hormonale

La fréquence optimale de régénération a été obtenue en testant 25 balances hormonales BAP 0,2 - 0,5 - 1 - 1,5 - 2 et ANA 0-0,5 - 0,05 observées pour 1,5 et 2 mg/l de BAP et 0,05 et 0,1 mg/l d'ANA. Le mélange 0,05 ANA et 2mg/l de BAP est retenu.

### 2. Autres dicotylédones

Les dicotylédones présentées dans le tableau 2 ont été transformées en utilisant la souche oncogène d'Agrobacterium A281 porteuse du cosmide pTVK291 associé au vecteur correspondant utilisant le matériel végétal rel qu'indiqué dans le tableau 2.

### B - Mesure de la résistance au bromoxynil

### 1.Tabac

La résistance a été mesurée in vitro par croissance de cals en présence de bromoxynil (20 mg/l) sous forme d'octonate et in vivo par pulvérisation foliaire avec du bromoxynil ou de l'ioxynil à des doses représentant 10 fois la dose recommandée pour un traitement en plein champ. Les résultats obtenus sont résumés dans le tableau 1bis.

### 2.Autres dicotylédones

La résistance a été mesurée in vitro par croissance de cals en présence de 10 mg de bromoxynil (sous forme d'octonate) par litre de milieu. Dans tous les cas les résultats sont positifs (tableau 2).

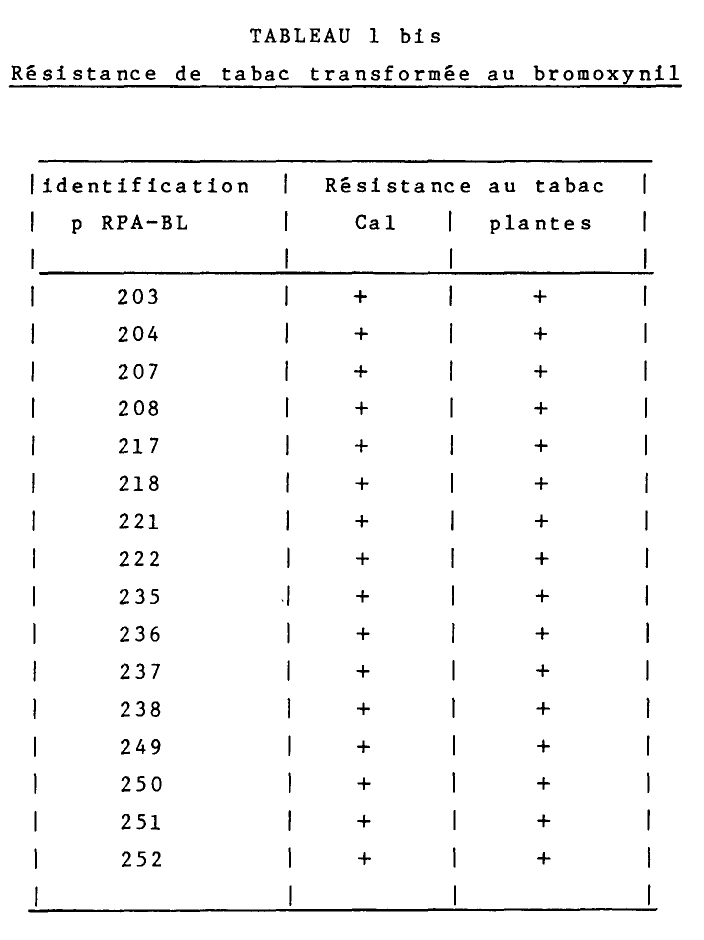

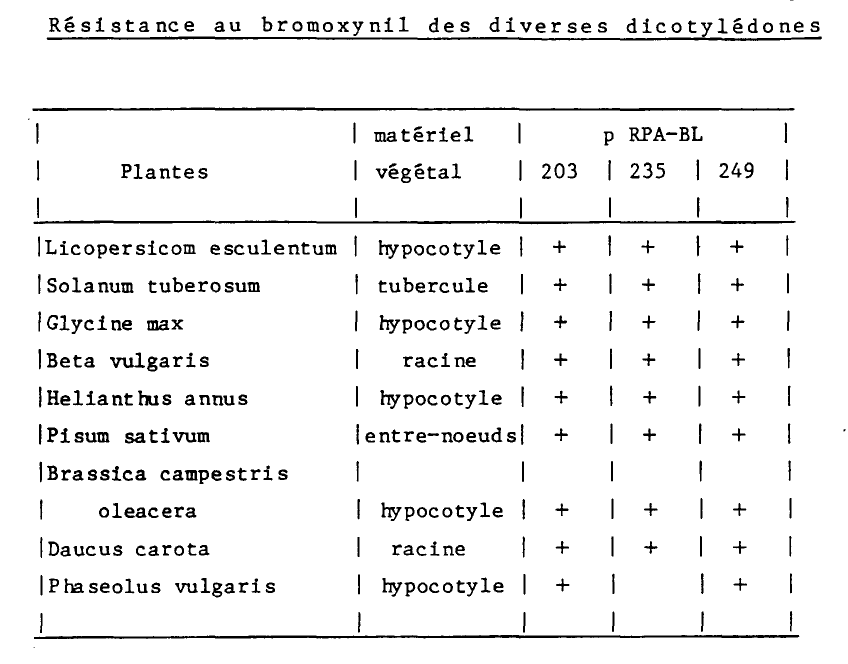

## Revendications

1. Gène chimérique utilisable pour conférer aux plantes une résistance à un herbicide à base de dihalogéno-3,5 hydroxy-4 benzonitrile comprenant au moins un gène codant pour la résistance à cet herbicide, un promoteur étranger et éventuellement une zone signal de polyadénylation, caractérisé en ce que le promoteur provient d'un gène s'exprimant naturellement dans des cellules végétales et choisi dans le groupe comprenant le promoteur de l'ARN 35S du virus de la mosaïque du chou-fleur (CamV 35S) et le promoteur de la petite sous unité (PSU) de la ribulose-1,5-bis phosphatocarboxylase oxygénase (RuBisCO de tournesol (Helianthus annuus)

2. Gène chimérique selon la revendication 1 caractérisé en ce qu'il comprend une zone intermédiaire non traduite (linker) entre le promoteur et le gène codant pour la résistance à l'herbicide, choisie dans le groupe comprenant la zone non traduite de pUC 19 modifié par clonage, la zone non traduite de la petite sous unité de la RuBisCO du maïs et la zone non traduite de la petite sous unité de la RuBisCO du tournesol.

3. Gène chimérique selon l'une des revendications 1 et 2, caractérisé en ce qu'il contient en outre, entre la zone intermédiaire et la zone codante pour la résistance à l'herbicide, une zone codante pour un peptide de transit capable de faire entrer la protéine dans le chloroplaste des plantes à transformer.

4. Gène chimérique selon le revendication 3, caractérisé en ce que la zone codante pour le peptide de transit est celle de la petite sous unité de la RuBisCO de maïs.

5. Gène chimérique selon le revendication 3, caractérisé en ce que la zone codante pour le peptide de transit est celle de la petite sous unité de la RuBisCO de tournesol.

6. Gène chimérique selon l'une des revendications 1 à 5, caractérisé en ce que la zone de polyadénylation provient du gène de la nopaline synthase.

7. Gène chimérique selon l'une des revendications 1 à 5, caractérisé en ce que la zone de polyadénylation provient du gène de la petite sous unité de la RuBisCO du maïs.

8. Gène chimérique selon l'une des revendications 1 à 7, caractérisé en ce que le gène code pour la résistance au bromoxynil ou à l'ioxynil ou l'un de leurs dérivés dels ou esters.

9. Vecteur utilisable pour la transformation de plantes en plantes résistantes à un herbicide à base de dihalogéno-3,5 hydroxy-4 benzonitrile, caractérisé en ce qu'il comprend un gène chimérique selon l'une des revendications 1 à 8.

10. Vecteur selon la revendication 9 caractérisé en qu'il est constitué d'un plasmide.

11. Microorganisme contenant un gène chimérique selon l'une des revendications 1 à 8.

12. Cellule végétale comportant un gène de résistance à un herbicide à base de dihalogéno-3,5 hydroxy-4 benzonitrile obtenue par intégration du gène selon l'une des revendications 1 à 8.

13. Plante ou partie de la plante transformée caractérisée en ce qu'elle est obtenue par régénération à partir de cellules végétales selon la revendication 12.

14. Semences de plantes transformées obtenues à partir de plantes selon la revendication 13.

15. Procédé de désherbage sélectif de plantes à l'aide d'un herbicide à base d'un dihalogéno-3,5 hydroxy-4 benzonitrile caractérisé en ce que l'herbicide est appliquée à une plante transformée selon l'une des revendications 13 et 14.

16. Utilisation du gène chimérique selon l'une des revendications 1 à 8 comme marqueur de sélection de cellules végétales, de cals, de partie de plantes ou de plantes.
